# EUROPEAN PATENT SPECIFICATION

(11) **EP 1 429 843 B1**
(45) Date of publication and mention of the grant of the patent: **27.12.2006**
(21) Application number: 02798730.4
(22) Date of filing: 17.09.2002
(51) Int. Cl.: A61P 11/06, A61K 31/47, A61K 31/44, A61K 45/06

(54) **COMBINATION OF A PDE INHIBITOR AND A LEUKOTRIENE RECEPTOR ANTAGONIST**
KOMBINATION VON EINEM PDE-HEMMER UND EINES LEUKOTRIEN REZEPTOR ANTAGONISTEN
COMBINAISON D'UN INHIBITEUR DE PDE ET D'UN ANTAGONISTE DU RECEPTEUR DE LEUKOTRIENE

(30) Priority: 19.09.2001 EP 01000474
(43) Date of publication of application: 23.06.2004
(73) Proprietor: ALTANA Pharma AG, 78467 Konstanz (DE)
(72) Inventor: BEUME, Rolf, 78465 Konstanz (DE); WEIMAR, Christian, 78467 Konstanz (DE); WOLLIN, Stefan-Lutz, 88709 Meersburg (DE)
(74) Representative: Wild, Robert
(86) International application number: PCT/EP2002/010423
(87) International publication number: WO 2003/024488

(56) References cited:
- WO-A-00/50011
- WO-A-01/13953
- WO-A-01/46151
- WO-A-01/57036
- WO-A-01/70738
- WO-A-01/90076
- KRAWIEC, MARZENA E. ET AL: "Leukotriene inhibitors and non-steroidal therapies in the treatment of asthma" EXPERT OPINION ON PHARMACOTHERAPY (2001), 2(1), 47-65, XP008002079
- CAMPILLO N. ET AL: "Novel bronchodilators in the treatment of asthma and COPD." EXPERT OPINION ON THERAPEUTIC PATENTS, (2002) 12/1 (53-63)., XP008002080

## Description

### Field of application of the invention

The present invention relates to combinations of pharmaceutically active substances for use in the treatment of respiratory tract disorders.

The substances used in the combinations according to the invention are known active compounds from the PDE4 inhibitors class and active compounds from the class of the leukotriene receptor antagonists.

### Known technical background

In the International Patent Application WO02/38155 the combined use of Roflumilast, Roflumilast-N-Oxide or a pharmaceutically acceptable salt of either compound on the one hand and of a leukotriene-receptor antagonist on the other hand for the treatment of bronchial and respiratory disorders is described. In the International Patent Application WO01/90076 a pharmaceutical composition comprising Roflumilast-N-Oxide and a leukotriene receptor antagonist is claimed. In the International Patent Application WO00/50011 sustained or controlled release formulations for PDE4 inhibitors are disclosed; in this document is furthermore mentioned that other drugs - inter alia leukotriene inhibitors - useful in treating PDE4 related disorders, can be incorporated in these formulations as well. In the International Patent Applications WO01/57036 and WO01/46151 PDE4 inhibitors of a certain formula are disclosed; both documents mention that the PDE4 inhibitors can be combined with leukotriene receptor antagonists. In Expert Opinion on Pharmacotherapy (2001), Vol. 2(1), pp. 47-65 Marzena E. Krawiec and Sally E. Wenzel describe Leukotriene inhibitors and several non-steroidal therapies in the treatment of asthma.

### Description of the invention

Asthma is a common inflammatory disease of the respiratory tract, accounting for 1 - 3% of all office visits, 500,000 hospital admissions per year and more pediatric hospital admissions than any other single illness in the US. Annually, more than 5000 children and adults die of asthma attacks in the United States (William E. S.; Goodmann Gilmann A.:The pharmacological Basis of Therapeutics, 9^{th} Edition, pp. 152 & 659-682, Mc Graw Hill, New York 1996).

Asthma can no longer be viewed simply as a reversible airway obstruction. It should instead be considered primarily as an inflammatory illness that has bronchial hyperactivity and bronchospasm as its results. Allergen specific immunoglobulin E (IgE) is bound to the mast cells via Fc receptors. It is a fragment obtained by papain digestion of immunoglobulin molecules and contains most of the antigenic determinants. When an allergen comes into contact with IgE, the mast cells are activated and release a number of inflammatory mediators, which include granule contents like histamine, proteases, heparin, and tumor necrosis factor (TNF), a variety of lipid membrane derived molecules like prostaglandins, leukotrienes and platelet activating factor (PAF), and a number of cytokines like interleukin (IL)-1, 3, 4, 5, 6 and 8 and chemokines. An enormous variety of mediators are released which have more than one potent effect on airway inflammation.

As a result of vasodilation, increased vasopermeability and increased endothelial adhesiveness towards leukocytes further leads to an influx of inflammatory cells like lymphocytes, eosinophils and macrophages from blood circulation into the tissues. This in turn leads to the release of mediators which have further inflammatory effects (Rao A. R. et al. Recent Perspectives in the design of antiasthmatic agents, Pharmazie, 55, 7, 475-482, 2000).

Thus, it can be understood that it is unlikely that drugs affecting a single mediator can satisfactorily treat the disease alone. As asthma is one of the major diseases affecting mankind, there is a need to develop drugs which can affect a wide variety of mediators.

Therefore, it is the object of the present invention to make available respiratory tract therapeutics which fulfil the following conditions:
- Favorable simultaneous influence on several of the inflammatory mediators
- Marked bronchorelaxation and -dilatation
- Good oral availability
- Minor side effects
- Good suitability for long-term therapy
- Favorable influence on bronchial hyperreactivity

It has now been found that the combined use of a PDE4 inhibitor and a leukotriene receptor antagonist outstandingly fulfills the above-mentioned conditions.

The invention thus relates to the combined use of a PDE4 inhibitor and a leukotriene receptor antagonist in the treatment of respiratory tract disorders.

"Combined use" in the context of the invention means the simultaneous, sequential or separate administration of the PDE4 inhibitor on the one hand and of the leukotriene receptor antagonist on the other hand.

Simultaneous administration includes - aside from the simultaneous uptake of two separate dosage forms containing the PDE4 inhibitor in the one and the leukotriene receptor antagonist in the other dosage form - pharmaceutical compositions containing both active ingredients in one single dosage form (fixed unit dose form).

Sequential administration in the context of the invention means the administration of the PDE4 inhibitor on the one hand and of the leukotriene receptor antagonist on the other hand in separate dosage forms within less than 12 hours, more preferably within less than one hour, most preferably within 5 minutes or less.

Separate administration within the context of the invention means the administration of the PDE4 inhibitor on the one hand and of the leukotriene receptor antagonist on the other hand in separate dosage forms within 12 hours or more.

"Combined use" in the context of the invention also includes a pharmaceutical product comprising both the PDE4 inhibitor and the leukotriene receptor antagonist as discrete separate dosage forms, in separate containers or e. g. in blisters containing both types of drugs in discrete solid dosage units, preferably in a form in which the dosage units which have to be taken together or which have to be taken within one day are grouped together in a manner which is convenient for the patient. Said pharmaceutical product itself or as a part of a kit may contain instructions for the simultaneous, sequential or separate administration of the discrete separate dosage units, to a patient in need thereof.

By the expression "PDE4 inhibitor" is meant a selective phosphodiesterase inhibitor, which inhibits preferentially the type 4 phosphodiesterase when compared to other known types of phosphodiesterase, e.g. type 1, 2, 3, 5 etc., whereby the compound has a lower IC₅₀ (more potent) for the type 4 phosphodiesterase, such as where the IC₅₀ for PDE4 inhibition is about factor 10 lower compared to IC₅₀ for inhibition of other known type of phosphodiesterase, e.g. type 1, 2, 3, 5 etc.

By the expression "leukotriene receptor antagonist" is meant leukotriene C4, leukotriene D4 and leukotriene E4 receptor antagonists, of which the leukotriene D4 receptor antagonists are preferred.

The PDE4 inhibitor is 3-Cyclopropylmethoxy-4-difluoromethoxy-N-(3,5-dichloropyrid-4-yl)-benzamide [INN: ROFLUMILAST].

The leukotriene receptor antagonist is 2-[1-[1(R)-[3-[2(E)-(7-chloroquinolin-2-yl)-vinyl]phenyl]-3-[2-(1-hydroxy-1-methylethyl)phenyl]propylsulfanylmethyl]cyclopropyl]acetic acid [INN: MONTELUKAST].

In the context of the present invention, unless otherwise stated, a pharmaceutically acceptable derivative of an active ingredient means a pharmaceutically acceptable salt or solvate (e. g. hydrate), a pharmaceutically acceptable solvate of such salt, a pharmaceutically acceptable N-oxide or a pharmaceutically acceptable salt or solvate of the latter.

Suitable pharmacologically tolerable salts here are on the one hand in particular water-soluble and water-insoluble acid addition salts with acids such as, for example, hydrochloric acid, hydrobromic acid, phosphoric acid, nitric acid, sulfuric acid, acetic acid, citric acid, D-gluconic acid, benzoic acid, 2-(4-hydroxybenzoyl)-benzoic acid, butyric acid, sulfosalicylic acid, maleic acid, lauric acid, malic acid, fumaric acid, succinic acid, oxalic acid, tartaric acid, embonic acid, stearic acid, toluenesulfonic acid, methanesulfonic acid or 1-hydroxy-2-naphthoic acid, the acids being employed in salt preparation - depending on whether it is a mono- or polybasic acid and depending on which salt is desired - in an equimolar quantitative ratio or one differing therefrom. Furthermore, the active compounds mentioned can also be present as pure enantiomers or as enantiomer mixtures in any mixing ratio.

On the other hand, salts with bases are also suitable. Examples of salts with bases which may be mentioned are alkali metal (lithium, sodium, potassium) or calcium, aluminum, magnesium, titanium, ammonium, meglumine or guanidinium salts, where here too the bases are employed in salt preparation in an equimolar quantitative ratio or one differing therefrom.

Certain of the active ingredients used in the present invention are capable of existing in stereoisomeric forms. The invention encompasses all stereoisomers of the active ingredients and mixtures thereof including racemates. Tautomers and mixtures thereof of the active ingredients are also part of the present invention.

In accordance with the present invention, there is provided in a first aspect a pharmaceutical composition comprising, in admixture, a first active ingredient which is selected from a PDE4 inhibitor and its pharmaceutically acceptable derivatives, and a second active ingredient which is selected from a leukotriene receptor antagonist and its pharmaceutically acceptable derivatives.

In a second aspect - which is an embodiment of the first aspect - there is provided a pharmaceutical composition comprising, in admixture, a first active ingredient which is selected from 3-Cyclopropylmethoxy-4-difluoromethoxy-N-(3,5-dichloropyrid-4-yl)-benzamide [INN: ROFLUMILAST] and its pharmaceutically acceptable derivatives, and a second active ingredient which is selected from 2-[1-[1 (R)-[3-[2(E)-(7-chloroquinolin-2-yl)vinyl]phenyl]-3-(2-(1-hydroxy-1-methylethyl)phenyl]propylsulfanylmethyl]cyclopropyl]acetic acid [INN: MONTELUKAST] and its pharmaceutically acceptable derivatives.

In a third aspect the invention provides a pharmaceutical product comprising, in combination, a preparation of a first active ingredient which is selected from a PDE4 inhibitor and its pharmaceutically acceptable derivatives, and a preparation of a second active ingredient which is selected from a leukotriene receptor antagonist and its pharmaceutically acceptable derivatives, for simultaneous, sequential or separate use in therapy.

In a fourth aspect - which is an embodiment of the third aspect - the invention provides a pharmaceutical product comprising, in combination, a preparation of a first active ingredient which is selected from 3-Cyclopropylmethoxy-4-difluoromethoxy-N-(3,5-dichloropyrid-4-yl)-benzamide [INN: ROFLUMILAST] and its pharmaceutically acceptable derivatives, and a preparation of a second active ingredient which is selected from 2-[1-[1(R)-[3-[2(E)-(7-chloroquinolin-2-yl)vinyl]phenyl]-3-[2-(1-hydroxy-1-methylethyl)-phenyl]propylsulfanylmethyl]cyclopropyl]acetic acid [INN: MONTELUKAST] and its pharmaceutically acceptable derivatives, for simultaneous, sequential or separate use in therapy.

In a fifth aspect, the invention provides a kit comprising a preparation of a first active ingredient which is selected from a PDE4 inhibitor and its pharmaceutically acceptable derivatives, a preparation of a second active ingredient which is selected from a leukotriene receptor antagonist and its pharmaceutically acceptable derivatives, and instructions for the simultaneous, sequential or separate administration of the preparations to a patient in need thereof.

In a sixth aspect - which is an embodiment of the fifth aspect - the invention provides a kit comprising a preparation of a first active ingredient which is selected from 3-Cyclopropylmethoxy-4-difluoromethoxy-N-(3,5-dichloropyrid-4-yl)-benzamide [INN: ROFLUMILAST] and its pharmaceutically acceptable derivatives, a preparation of a second active ingredient which is selected from 2-[1-[1 (R)-[3-[2(E)-(7-chloroquinolin-2-yl)vinyl]phenyl]-3-[2-(1-hydroxy-1-methylethyl)phenyl]propylsulfanylmethyl]cyclopropyl]acetic acid [INN: MONTELUKAST] and its pharmaceutically acceptable derivatives, and instructtions for the simultaneous, sequential or separate administration of the preparations to a patient in need thereof.

It has been found that the administration of active ingredients according to the invention is advantageous because it results - in comparison to the administration of a single active ingredient from the PDE4 inhibitors or the leukotriene receptor antagonists class - in a reduced early allergic response as well as in a reduced late inflammatory airway response.

The pharmaceutical composition of the present invention may be prepared by mixing the first active ingredient with the second active ingredient.

In the above-mentioned mixing process the first active ingredient and the second active ingredient can
a) in a first step be mixed as such, afterwards be processed with pharmaceutically acceptable auxiliaries and/or excipients and finally pressed to tablets or caplets
   or
b) in a first step separately be processed with pharmaceutically acceptable auxiliaries and/or excipients to give granules or pellets containing each only one of the two active ingredients; the pellets or granules for their part then can be mixed in an appropriate ratio and either be pressed - optionally with further pharmaceutically acceptable auxiliaries and/or excipients - to give for example, tablets or caplets, or can be filled in more or less loose form in capsules.

Therefore, in a seventh aspect of the present invention, there is provided a process for the preparation of a pharmaceutical composition which comprises mixing a first active ingredient which is selected from a PDE4 inhibitor and its pharmacologically acceptable derivatives, with a second active ingredient which is selected from a leukotriene receptor antagonist and its pharmacologically acceptable derivatives.

In an eighth aspect - which is an embodiment of the seventh aspect - there is provided a process for the preparation of a pharmaceutical composition which comprises mixing a first active ingredient which is selected from 3-Cyclopropylmethoxy-4-difluoromethoxy-N-(3,5-dichloropyrid-4-yl)-benzamide [INN: ROFLUMILAST] and its pharmacologically acceptable derivatives, with a second active ingredient which is selected from 2-[1-[1(R)-[3-[2(E)-(7-chloroquinolin-2-yl)vinyl]phenyl]-3-[2-(1-hydroxy-1-methylethyl)phenyl]propylsulfanylmethyl]cyclopropyl]acetic acid [INN: MONTELUKAST] and its pharmacologically acceptable derivatives.

The present invention further provides the use of a pharmaceutical composition, or pharmaceutical product according to the invention in the manufacture of a medicament for the prophylaxis and/or treatment of a respiratory tract disorder.

Respiratory tract disorders which may be mentioned are in particular allergen- and inflammation-induced bronchial disorders (bronchitis, obstructive bronchitis, spastic bronchitis, allergic bronchitis, allergic asthma, bronchial asthma, COPD, allergic, seasonal and perennial rhinitis), which can be treated by the combination according to the invention also in the sense of a long-term therapy (if desired with appropriate adjustment of the dosage of the individual components to the needs at the time, for example needs subject to seasonally related variations).

The active ingredients may, and indeed will, as part of the pharmaceutical composition, the pharmaceutical product or preparation, be used in admixture with one or more pharmaceutically acceptable auxiliaries and/or excipients.

The person skilled in the art is familiar on the basis of his/her expert knowledge with which excipients or auxiliaries are suitable for the desired pharmaceutical composition, product or preparation. In addition to solvents, gel-forming agents, tablet excipients and other active compound carriers, it is possible to use, for example, antioxidants, dispersants, emulsifiers, antifoams, flavor corrigents, preservatives, solubilizers, colorants or permeation promoters and complexing agents (e.g. cyclodextrins).

Within the meaning of the present invention, "use" is preferably understood as meaning the oral administration of both active ingredients. Further methods of administration, which may be mentioned are the parenteral, intranasal, sublingual or rectal administration of the active ingredients. The active ingredients may as well be administrated by inhalation or insufflation.

The pharmaceutical compositions or preparations according to the invention are preferably in unit dosage form such as tablets, coated tablets, pills, capsules, caplets, powders, granules, emulsions, suspensions or (sterile parenteral) solutions, metered aerosol or liquid sprays, drops ampoules transdermal patches, auto-injector devices or suppositories; the active ingredient content advantageously being between 0.1 and 95% and by appropriate choice of the excipients and the auxiliaries, it being possible to achieve a pharmaceutical administration form precisely tailored to the active ingredient(s) and/or to the desired onset of action (e.g. a sustained release form or an enteric form).

For the above-mentioned therapeutic uses the dosages administered will, of course, vary with the first and second active ingredients employed, the mode of administration, the treatment desired and the disorder indicated.

However, in general, satisfactory results will be obtained when the total daily dosage of first active ingredient, the PDE4 inhibitor ROFLUMILAST, when taken orally is in the range from 1-20 µg/kg of body weight.

The total daily dosage of the second active ingredient, the leukotriene receptor antagonist MONTELUKAST, when taken orally is in a range from 50-400 µg/kg of body weight.

### Pharmacology

### Objective:

To assess the additive or synergistic inhibitory effect of the selective phosphodiesterase-4 inhibitor ROFLUMILAST combined with the leukotriene receptor antagonist MONTELUKAST both orally administered 1 h before ovalbumin (OVA) challenge on the early, mainly leukotriene-mediated (SRS-A = slow reacting substance of anaphylaxis) bronchoconstriction in anaesthetized, mechanically ventilated guinea pigs.

### Animals:

Male Dunkin Hartley guinea pigs; body weight 200-250 g at sensitization and 350-500 g when performing the experiments.

### Drugs, substances and experimental procedure:

Ovalbumin (OVA) was used as allergen for active sensitization. Therefore, 20 µg OVA together with 20 mg Al(OH)₃ suspended in 0.5 ml 0.9%NaCl-solution (= saline) were administered i.p. to each animal on 2 consecutive days. After 2-3 weeks the animals were challenged with a single i.v. dose of 0.15 mg/kg OA suspended in saline. Drugs were suspended in a 4%-methocel-solution and administered p.o. by gavage to the conscious animals (n = 10 animals per dose) 1 h before OVA-challenge:
1) ROFLUMILAST (3-cyclopropylmethoxy-4-difluoromethoxy-N-(3,5-dichloropyrid-4-yl)-benzamide) at doses of 0.01, 0.1, 1.0, and 3.0 µmol/kg (0.004, 0.04, 0.4, and 1.2 mg/kg)
2) MONTELUKAST sodium salt 2-[1-[1(R)-[3-[2(E)-(7-chloroquinolin-2-yl)vinyl]phenyl]-3-[2-(1-hydroxy-1-methylethyl)phenyl]propylsulfanylmethyl]cyclopropyl]acetic acid sodium salt) at doses of 0.03, 0.10, 0.17, and 0.5 µmol/kg (0.018, 0.06, 0.1, and 0.3 mg/kg)
3) ROFLUMILAST at doses of 0.1, 0.3, and 1 µmol/kg (0.04, 0.12, and 0.4 mg/kg) in combination with MONTELUKAST sodium salt 0.03 µmol/kg (0.018 mg/kg)

Controls received the drug-free methocel-solution 1 h before OVA-challenge as placebo (n = 12). OVA-challenge and lung function measurements were performed in anaesthetized and mechanically ventilated animals. Urethane was used for anesthesia and was injected i.p. at a dose of 1.5 g/kg as 12.5 % solution about 50 min before OVA-challenge. To abolish spontaneous breathing, animals received 1.5 mg/kg i.v. of the muscle relaxant pancuronium bromide about 14 min before OVA-challenge. To enhance the SRS-A-mediated bronchoconstriction, the animals were pretreated with: Indomethacin 10 mg/kg i.v. 20 min before OVA-challenge to block the cyclooxygenase pathway and to enhance the production of lipoxygenase-mediators, mainly leukotrienes. Pyrilamine 2 mg/kg i.v. 6 min before OVA-challenge to inhibit the bronchoconstrictor effect of endogenously liberated histamine. Propranolol 0.1 mg/kg i.v. 5 min before OVA-challenge to abolish endogenous adrenergic tonus, Administration volume was 1 ml/kg for all i.v. injected compounds.

### Measurement of the SRS-A-mediated bronchoconstriction:

After injection of the muscle relaxant, the animals were ventilated at 60 breaths/min, 7 ml/kg tidal volume and 40/60% inspiration/expiration ratio using a small animal Ventilator. Dynamic lung compliance and airway conductance (=1/resistance) were calculated breath-by-breath from tidal volume and pulmonary inflation pressure and from flow and pulmonary inflation pressure, respectively, using a PC-based software program for analysis, data acquisition and archiving of the respiratory and cardiovascular parameters. The SRS-A-mediated bronchoconstriction was induced by i.v. injection of OVA. This was characterized by a decrease of compliance and conductance, which started about 1 min after OVA-injection and reached a plateau at 70-90% decrease after 4-6 min. The time-effect curve for the delta-%-decrease of compliance and conductance was determined up to 12 min post challenge. Blood pressure and heart rate were monitored to control vitality.

### Data analysis:

The area under the time-delta-%-decrease curves (AUC) was determined for each animal. On basis of the AUC-values, the inhibition of the SRS-A-induced decrease of compliance and conductance was calculated in reference to placebo. After eliminating drop-outs (Grubb's-test), dose-response curves were established, principally based on log-linear regression analysis, to determine ED₅₀-values. Pharmacodynamic effect of ROFLUMILAST combined with MONTELUKAST sodium salt was measured and compared with the calculated effects according to G. Pöch [1]. Measured and calculated ED₅₀-values were compared by an unpaired t-test for statistical significant differences.

### Results:

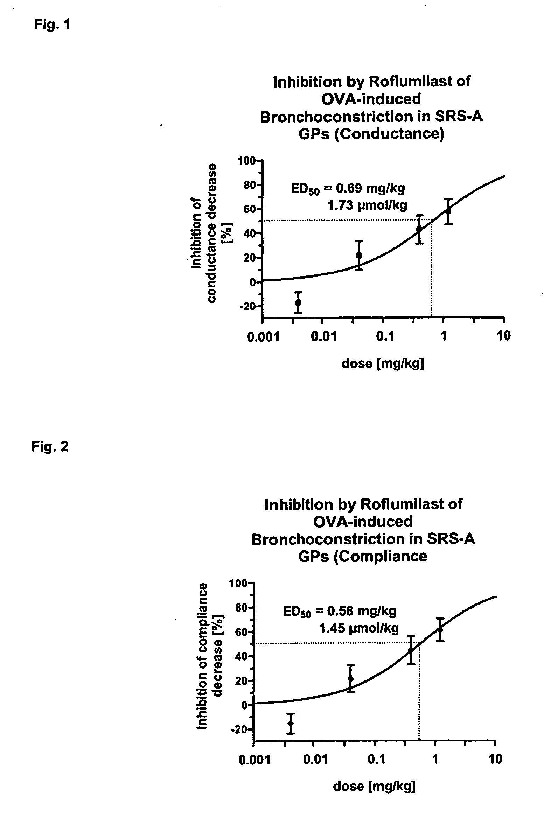

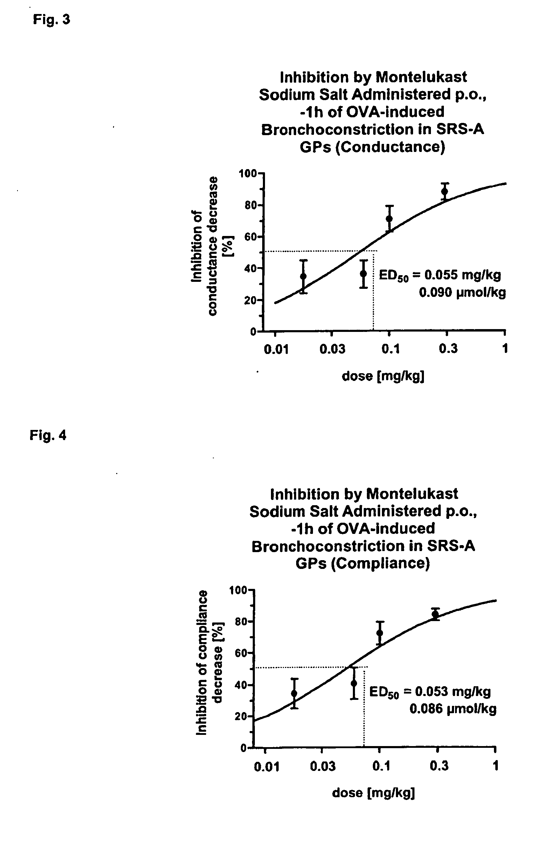

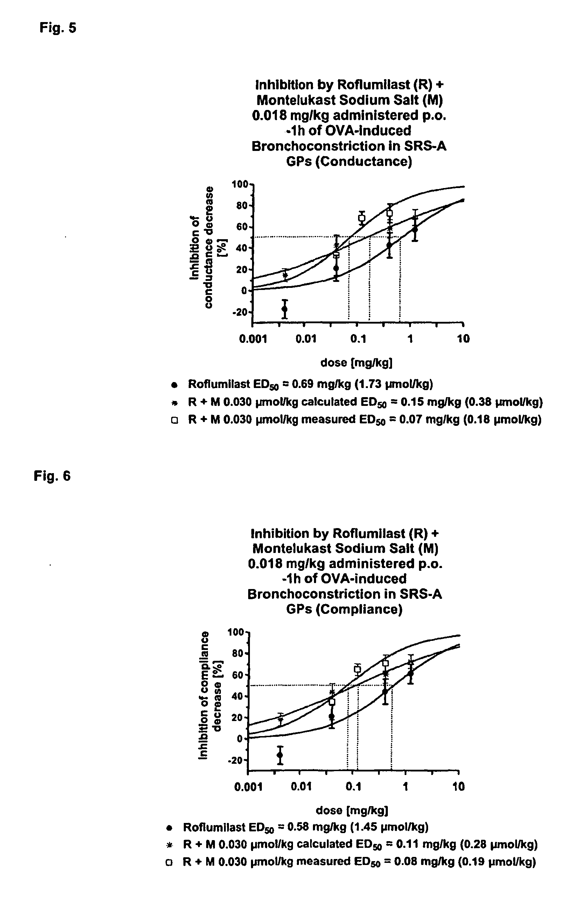

1) ROFLUMILAST inhibited decrease of airway conductance with an ED₅₀ of 0.69 mg/kg (1.73 µmol/kg) and airway compliance with an ED₅₀ of 0.58 µmol/kg (1.45 mg/kg) (Fig.1 and 2).
2) MONTELUKAST sodium salt inhibited decrease of airway conductance with an ED₅₀ of 0.055 mg/kg (0.090 µmol/kg) and airway compliance with an ED₅₀ of 0.053 µmol/kg (0.086 mg/kg) (Fig. 3 and 4).
3) ROFLUMILAST combined with MONTELUKAST sodium salt 0.018 mg/kg (0.030 µmol/kg) inhibited decrease of airway conductance with an ED₅₀ of 0.07 mg/kg (0.18 µmol/kg) and airway compliance with an ED₅₀ of 0.08 µmol/kg (0.19 mg/kg) respectively. (Fig. 5 and 6)
4) If one calculates the ED₅₀ values according to the method described by G. Pöch [1] for ROFLUMILAST combined with MONTELUKAST sodium salt 0.018 mg/kg (0.030 µmol/kg) on the basis of the values given in 1) and 2) the ED₅₀ values come out as follows: ED₅₀ = 0.15 mg/kg (0.38 µmol/kg) for airway conductance and ED₅₀ = 0.11 (0.28 µmol/kg) for airway compliance (Fig. 5 and 6).
5) Measured ED₅₀s are not significantly different from calculated ED₅₀s.

### Summary:

Inhibitory effects of ROFLUMILAST and MONTELUKAST sodium salt on SRS-A-induced bronchoconstriction are additive.

### References:

G. Pöch. Quantitative Ermittlung potenzierender oder hemmender Kombinations-wirkungen gleichsinnig wirkender Pharmaka. 1981, Arzneim. Forsch. / Drug Res. 31 (II), No. 7, 1135-1140

### Description of the Figures:

Figure 1: Inhibition by ROFLUMILAST of OVA-induced Bronchoconstriction in SRS-A GPs (Conductance)
Figure 2: Inhibition by ROFLUMILAST of OVA-induced Bronchoconstriction in SRS-A GPs (Compliance)
Figure 3: Inhibition by MONTELUKAST sodium salt administered p.o. -1h of OVA-induced Bronchoconstriction in SRS-A GPs (Conductance)
Figure 4: Inhibition by MONTELUKAST sodium salt administered p.o. -1h of OVA-induced Bronchoconstriction in SRS-A GPs (Compliance)
Figure 5: Inhibition by ROFLUMILAST + MONTELUKAST sodium salt 0.018 mg/kg administered p.o. -1h of OVA-induced Bronchoconstriction in SRS-A GPs (Conductance)
Figure 6: Inhibition by ROFLUMILAST + MONTELUKAST sodium salt 0.018 mg/kg administered p.o. -1h of OVA-induced Bronchoconstriction in SRS-A GPs (Compliance)

## Claims

1. A pharmaceutical composition comprising, in admixture, a first active ingredient which is a PDE4 inhibitor selected from 3-Cyclopropylmethoxy-4-difluoromethoxy-N-(3,5-dichloropyrid-4-yl)-benzamide [INN: Roflumilast] and a pharmaceutically acceptable salt, solvate, N-Oxide or solvate of a salt or N-oxide thereof, and a second active ingredient which is a leukotriene receptor antagonist selected from 2-[1-[1(R)-[3-[2(E)-(7-chloroquinolin-2-yl)vinyl]phenyl]-3-[2-(1-hydroxy-1-methylethyl)phenyl]propylsulfanylmethyl]cyclopropyl]acetic acid [INN: MONTELUKAST] and a pharmaceutically acceptable salt, solvate, N-Oxide or solvate of a salt or N-oxide thereof.

2. A pharmaceutical composition comprising, in admixture, a first active ingredient which is 3-Cyclopropylmethoxy-4-difluoromethoxy-N-(3,5-dichloropyrid-4-yl)-benzamide [INN: Roflumilast], and a second active ingredient which is the sodium salt of 2-[1-[1(R)-[3-[2(E)-(7-chloroquinolin-2-yl)vinyl]phenyl]-3-[2-(1-hydroxy-1-methylethyl)phenyl]propylsulfanylmethyl]cyclopropyl]acetic acid [INN: MONTELUKAST].

3. A pharmaceutical composition according to any one of claims 1 or 2, which is a fixed oral combination.

4. A process for the preparation of a pharmaceutical composition as defined in any one of claims 1, 2 or 3 which comprises mixing the first active ingredient with the second active ingredient.

5. A pharmaceutical product comprising, in combination, a preparation of a first active ingredient which is a PDE4 inhibitor selected from 3-Cyclopropylmethoxy-4-difluoromethoxy-N-(3,5-dichloropyrid-4-yl)-benzamide [INN: Roflumilast] and a pharmaceutically acceptable salt, solvate, N-Oxide or solvate of a salt or N-oxide thereof and a preparation of a second active ingredient which is a leukotriene receptor antagonist selected from 2-[1-[1 (R)-[3-[2(E)-(7-chloroquinolin-2-yl)vinyl]phenyl]-3-[2-(1-hydroxy-1-methylethyl)phenyl]propylsulfanylmethyl]cyclopropyl]acetic acid [INN: MONTELUKAST] and a pharmaceutically acceptable salt, solvate, N-Oxide or solvate of a salt or N-oxide thereof, for simultaneous, sequential or separate use in therapy.

6. A pharmaceutical product comprising, in combination, a preparation of a first active ingredient which is 3-Cyclopropylmethoxy-4-difluoromethoxy-N-(3,5-dichloropyrid-4-yl)-benzamide [INN: Roflumilast] and a preparation of a second active ingredient which is the sodium salt of 2-[1-[1(R)-[3-[2(E)-(7-chloroquinolin-2-yl)vinyl]phenyl]-3-[2-(1-hydroxy-1-methylethyl)phenyl]propylsulfanylmethyl]cyclopropyl]acetic acid [INN: MONTELUKAST] for simultaneous, sequential or separate use in therapy.

7. A kit comprising a preparation of a first active ingredient, which is a PDE4 inhibitor selected from 3-Cyclopropylmethoxy-4-difluoromethoxy-N-(3,5-dichloropyrid-4-yl)-benzamide [INN: Roflumilast] and a pharmaceutically acceptable salt, solvate, N-Oxide or solvate of a salt or N-oxide thereof, a preparation of a second active ingredient which is a leukotriene receptor antagonist selected from 2-[1-[1(R)-[3-[2(E)-(7-chloroquinolin-2-yl)vinyl]phenyl]-3-[2-(1-hydroxy-1-methylethyl)phenyl]propylsulfanylmethyl]cyclopropyl]acetic acid [INN: MONTELUKAST] and a pharmaceutically acceptable salt, solvate, N-Oxide or solvate of a salt or N-oxide thereof, and instructions for simultaneous, sequential or separate administration of the preparations to the patient in need thereof.

8. A kit comprising a preparation of a first active ingredient, which is 3-Cyclopropylmethoxy-4-difluoromethoxy-N-(3,5-dichloropyrid-4-yl)-benzamide [INN: Roflumilast], a preparation of a second active ingredient which is the sodium salt of 2-[1-[1(R)-[3-[2(E)-(7-chloroquinolin-2-yl)vinyl]phenyl]-3-[2-(1-hydroxy-1-methylethyl)phenyl]propylsulfanylmethyl]cyclopropyl]acetic acid [INN: MONTELUKAST], and instructions for simultaneous, sequential or separate administration of the preparations to the patient in need thereof.

9. Use of a pharmaceutical composition according to any one of claims 1, 2 or 3 in the manufacture of a medicament for the treatment of a respiratory tract disorder.

10. Use according to claim 9, wherein the respiratory tract disorder is selected from bronchitis, obstructive bronchitis, spastic bronchitis, allergic bronchitis, allergic asthma, bronchial asthma, COPD, allergic rhinitis, seasonal rhinitis or perennial rhinitis.

11. Use according to claim 9, wherein the respiratory tract disorder is selected from allergic asthma, bronchial asthma and allergic rhinitis.

12. Use of a pharmaceutical product according to any one of claims 5 or 6 in the manufacture of a medicament for the treatment of a respiratory tract disorder.

13. Use according to claim 12, wherein the respiratory tract disorder is selected from bronchitis, obstructive bronchitis, spastic bronchitis, allergic bronchitis, allergic asthma, bronchial asthma, COPD, allergic rhinitis, seasonal rhinitis or perennial rhinitis.

14. Use according to claim 12, wherein the respiratory tract disorder is selected from allergic asthma, bronchial asthma and allergic rhinitis.

## Patentansprüche

1. Pharmazeutische Zusammensetzung enthaltend, als Mischung, einen ersten Wirkstoff, bei dem es sich um einen PDE4-Inhibitor ausgewählt aus 3-Cyclopropylmethoxy-4-difluormethoxy-N-(3,5-dichlorpyrid-4-yl)-benzamid [INN: Roflumilast] und einem pharmazeutisch unbedenklichen Salz, Solvat, N-Oxid oder Solvat eines Salzes oder N-Oxids davon handelt, und einen zweiten Wirkstoff, bei dem es sich um einen Leukotrienrezeptorantagonisten ausgewählt aus 2-[1-[1(R)-[3-[2(E)-(7-Chlorchinolin-2-yl)vinyl]phenyl]-3-(2-(1-hydroxy-1-methylethyl)phenyl]propylsulfanylmethyl]cyclopropyl]essigsäure [INN: MONTELUKAST] und einem pharmazeutisch unbedenklichen Salz, Solvat, N-Oxid oder Solvat eines Salzes oder N-Oxids davon handelt.

2. Pharmazeutische Zusammensetzung enthaltend, als Mischung, einen ersten Wirkstoff, bei dem es sich um 3-Cyclopropylmethoxy-4-difluormethoxy-N-(3,5-dichlorpyrid-4-yl)-benzamid [INN: Roflumilast], und einen zweiten Wirkstoff, bei dem es sich um das Natriumsalz von 2-[1-[1(R)-[3-[2(E)-(7-Chlorchinolin-2-yl)vinyl]phenyl]-3-[2-(1-hydroxy-1-methylethyl)phenyl]propylsulfanylmethyl]cyclopropyl]essigsäure [INN: MONTELUKAST] handelt.

3. Pharmazeutische Zusammensetzung nach Anspruch 1 oder 2, bei der es sich um eine festgelegte orale Kombination handelt.

4. Verfahren zur Herstellung einer pharmazeutischen Zusammensetzung nach einem der Ansprüche 1, 2 oder 3, bei dem man den ersten Wirkstoff mit dem zweiten Wirkstoff mischt.

5. Pharmazeutisches Produkt enthaltend, als Kombination, eine Zubereitung eines ersten Wirkstoffs, bei dem es sich um einen PDE4-Inhibitor ausgewählt aus 3-Cyclopropylmethoxy-4-difluormethoxy-N-(3,5-dichlorpyrid-4-yl)-benzamid [INN: Roflumilast] und einem pharmazeutisch unbedenklichen Salz, Solvat, N-Oxid oder Solvat eines Salzes oder N-Oxids davon handelt, und eine Zubereitung eines zweiten Wirkstoffs, bei dem es sich um einen Leukotrienrezeptorantagonisten ausgewählt aus 2-[1-[1(R)-[3-[2(E)-(7-Chlorchinolin-2-yl)vinyl]phenyl]-3-[2-(1-hydroxy-1-methylethyl)phenyl]propylsulfanylmethyl]cyclopropyl]essigsäure [INN: MONTELUKAST] und einem pharmazeutisch unbedenklichen Salz, Solvat, N-Oxid oder Solvat eines Salzes oder N-Oxids davon handelt, zur gleichzeitigen, aufeinanderfolgenden oder getrennten Verwendung in der Therapie.

6. Pharmazeutisches Produkt enthaltend, als Kombination, eine Zubereitung eines ersten Wirkstoffs, bei dem es sich um 3-Cyclopropylmethoxy-4-difluormethoxy-N-(3,5-dichlorpyrid-4-yl)-benzamid [INN: Roflumilast] handelt, und eine Zubereitung eines zweiten Wirkstoffs, bei dem es sich um das Natriumsalz von 2-[1-[1(R)-[3-[2(E)-(7-Chlorchinolin-2-yl)vinyl]phenyl]-3-[2-(1-hydroxy-1-methylethyl)phenyl]propylsulfanylmethyl]cyclopropyI]essigsäure [INN: MONTELUKAST] handelt, zur gleichzeitigen, aufeinanderfolgenden oder getrennten Verwendung in der Therapie.

7. Kit, enthaltend eine Zubereitung eines ersten Wirkstoffs, bei dem es sich um einen PDE4-Inhibitor ausgewählt aus 3-Cyclopropylmethoxy-4-difluormethoxy-N-(3,5-dichlorpyrid-4-yl)-benzamid [INN: Roflumilast] und einem pharmazeutisch unbedenklichen Salz, Solvat, N-Oxid oder Solvat eines Salzes oder N-Oxids davon handelt, eine Zubereitung eines zweiten Wirkstoffs, bei dem es sich um einen Leukotrienrezeptorantagonisten ausgewählt aus 2-[1-[1(R)-[3-[2(E)-(7-Chlorchinolin-2-yl)vinyl]phenyl]-3-[2-(1-hydroxy-1-methylethyl]phenyl]propylsulfanylmethyl]cyclopropyl]essigsäure [INN: MONTELUKAST] und einem pharmazeutisch unbedenklichen Salz, Solvat, N-Oxid oder Solvat eines Salzes oder N-Oxids davon handelt, und Anweisungen für die gleichzeitige, aufeinanderfolgende oder getrennte Verabreichung der Zubereitungen an den bedürftigen Patienten.

8. Kit, enthaltend eine Zubereitung eines ersten Wirkstoffs, bei dem es sich um 3-Cyclopropylmethoxy-4-difluormethoxy-N-(3,5-dichlorpyrid-4-yl)-benzamid [INN: Roflumilast] handelt, eine Zubereitung eines zweiten Wirkstoffs, bei dem es sich um das Natriumsalz von 2-[1-[1(R)-[3-[2(E)-(7-Chlorchinolin-2-yl)vinyl]phenyl]-3-[2-(1-hydroxy-1-methylethyl)phenyl]propylsulfanylmethyl]-cyclopropyl]essigsäure [INN: MONTELUKAST] handelt, und Anweisungen für die gleichzeitige, aufeinanderfolgende oder getrennte Verabreichung der Zubereitungen an den bedürftigen Patienten.

9. Verwendung einer pharmazeutischen Zusammensetzung nach einem der Ansprüche 1, 2 oder 3, bei der Herstellung eines Medikaments zur Behandlung einer Atemwegserkrankung.

10. Verwendung nach Anspruch 9, wobei die Atemwegserkrankung ausgewählt ist aus Bronchitis, obstruktiver Bronchitis, spastischer Bronchitis, allergischer Bronchitis, allergischem Asthma, Bronchialasthma, chronisch-obstruktiver Atemwegserkrankung (COPD), allergischer Rhinitis, saisonaler Rhinitis oder perennialer Rhinitis.

11. Verwendung nach Anspruch 9, wobei die Atemwegserkrankung ausgewählt ist aus allergischem Asthma, Bronchialasthma und allergischer Rhinitis.

12. Verwendung eines pharmazeutischen Produkts nach Anspruch 5 oder 6 bei der Herstellung des Medikaments zur Behandlung einer Atemwegserkrankung.

13. Verwendung nach Anspruch 12, wobei die Atemwegserkrankung ausgewählt ist aus Bronchitis, obstruktiver Bronchitis, spastischer Bronchitis, allergischer Bronchitis, allergischem Asthma, Bronchialasthma, chronisch-obstruktiver Atemwegserkrankung (COPD), allergischer Rhinitis, saisonaler Rhinitis oder perennialer Rhinitis.

14. Verwendung nach Anspruch 12, wobei die Atemwegserkrankung ausgewählt ist aus allergischem Asthma, Bronchialasthma und allergischer Rhinitis.

## Revendications

1. Composition pharmaceutique comprenant, en mélange, un premier principe actif qui est un inhibiteur de PDE4, choisi parmi le 3-cyclopropylméthoxy-4-difluorométhoxy-N-(3,5-dichloropyrid-4-yl)-benzamide [DCI : Roflumilast] et un sel, solvate, N-oxyde ou solvate d'un sel ou d'un N-oxyde pharmaceutiquement acceptable de celui-ci et un deuxième principe actif qui est un antagoniste des récepteurs des leucotriènes, choisi parmi l'acide 2-[1-[1(R)-[3-[2(E)-(7-chloroquinoléin-2-yl)vinyl]phényl]-3-[2-(1-hydroxy-1-méthyléthyl)phényl]propylsulfanylméthyl]cyclopropyl] acétique [DCI : MONTELUKAST] et un sel, solvate, N-oxyde ou solvate d'un sel ou d'un N-oxyde pharmaceutiquement acceptable de celui-ci.

2. Composition pharmaceutique comprenant, en mélange, un premier principe actif qui est le 3-cyclopropylméthoxy-4-difluorométhoxy-N-(3,5-dichloropyrid-4-yl)-benzamide [DCI : Roflumilast] et un deuxième principe actif qui est le sel sodique de l'acide 2-[1-[1 (R)-[3-[2(E)-(7-chloroquinoléin-2-yl)vinyl]phényl]-3-[2-(1-hydroxy-1-méthyléthyl)phényl]propylsulfanylméthyl]cyclopropyl]-acétique [DCI : MONTELUKAST].

3. Composition pharmaceutique selon l'une quelconque des revendications 1 ou 2, qui est une combinaison fixe orale.

4. Procédé de préparation d'une composition pharmaceutique telle que définie dans l'une quelconque des revendications 1, 2 ou 3, qui comprend le mélange du premier principe actif avec le deuxième principe actif.

5. Produit pharmaceutique comprenant, en combinaison, une préparation d'un premier principe actif qui est un inhibiteur de PDE4, choisi parmi le 3-cyclopropylméthoxy-4-difluorométhoxy-N-(3,5-dichloropyrid-4-yl)-benzamide [DCI : Roflumilast] et un sel, solvate, N-oxyde ou solvate d'un sel ou d'un N-oxyde pharmaceutiquement acceptable de celui-ci et une préparation d'un deuxième principe actif qui est un antagoniste des récepteurs des leucotriènes, choisi parmi l'acide 2-[1-[1(R)-[3-[2(E)-(7-chloroquinoléin-2-yl)yinyl]phényl]-3-[2-(1-hydroxy-1-méthyléthyl)-phényl]propylsulfanylméthyl]cyclopropyl] acétique [DCI : MONTELUKAST] et un sel, solvate, N-oxyde ou solvate d'un sel ou d'un N-oxyde pharmaceutiquement acceptable de celui-ci, destiné à une utilisation simultanée, séquentielle ou séparée en thérapie.

6. Produit pharmaceutique comprenant, en combinaison, une préparation d'un premier principe actif qui est le 3-cyclopropylméthoxy-4-difluorométhoxy-N-(3,5-dichloropyrid-4-yl)-benzamide [DCI : Roflumilast] et une préparation d'un deuxième principe actif qui est le sel sodique de l'acide 2-[1-[1(R)-[3-[2(E)-(7-chloroquinoléin-2-yl)vinyl]phényl]-3-[2-(1-hydroxy-1-méthyléthyl)phényl]-propylsulfanylméthyl]cyclopropyl] acétique [DCI : MONTELUKAST], destiné à une utilisation simultanée, séquentielle ou séparée en thérapie.

7. Kit comprenant une préparation d'un premier principe actif qui est un inhibiteur de PDE4, choisi parmi le 3-cyclopropylméthoxy-4-difluorométhoxy-N-(3,5-dichloropyrid-4-yl)-benzamide [DCI : Roflumilast] et un sel, solvate, N-oxyde ou solvate d'un sel ou d'un N-oxyde pharmaceutiquement acceptable de celui-ci et une préparation d'un deuxième principe actif qui est un antagoniste des récepteurs des leucotriènes, choisi parmi l'acide 2-[1-[1(R)-[3-[2(E)-(7-chloroquinoléin-2-yl)vinyl]phényl]-3-{2-(1-hydroxy-1-méthyléthyl)phényl]propylsulfanylméthyl]cyclopropyl]acétique[DCI: MONTELUKAST] et un sel, solvate, N-oxyde ou solvate d'un sel ou d'un N-oxyde pharmaceutiquement acceptable de celui-ci, et des instructions pour une administration simultanée, séquentielle ou séparée des préparations au patient en ayant besoin.

8. Kit comprenant une préparation d'un premier principe actif qui est le 3-cyclopropylméthoxy-4-difluorométhoxy-N-(3,5-dichloropyrid-4-yl)-benzamide [DCI : Roflumilast], une préparation d'un deuxième principe actif qui est le sel sodique de l'acide 2-[1-[1(R)-[3-[2(E)-(7-chloroquinoléin-2-yl)vinyl]phényl]-3-{2-( 1-hydroxy-1-méthyléthyl)phényl]propylsulfanylméthyl]cyclopropyl]acétique [DCI : MONTELUKAST] et des instructions pour une administration simultanée, séquentielle ou séparée des préparations au patient en ayant besoin.

9. Utilisation d'une composition pharmaceutique selon l'une quelconque des revendications 1, 2 ou 3 dans la fabrication d'un médicament destiné au traitement d'un trouble des voies respiratoires.

10. Utilisation selon la revendication 9, **caractérisée en ce que** le trouble des voies respiratoires est choisi parmi la bronchite, la bronchite obstructive, la bronchite spastique, la bronchite allergique, l'asthme allergique, l'asthme bronchique, la BPCO, la rhinite allergique, la rhinite saisonnière et la rhinite perannuelle.

11. Utilisation selon la revendication 9, **caractérisée en ce que** le trouble des voies respiratoires est choisi parmi l'asthme allergique, l'asthme bronchique et la rhinite allergique.

12. Utilisation d'un produit pharmaceutique selon l'une quelconque des revendications 5 ou 6 dans la fabrication d'un médicament destiné au traitement d'un trouble des voies respiratoires.

13. Utilisation selon la revendication 12, **caractérisée en ce que** le trouble des voies respiratoires est choisi parmi la bronchite, la bronchite obstructive, la bronchite spastique, la bronchite allergique, l'asthme allergique, l'asthme bronchique, la BPCO, la rhinite allergique, la rhinite saisonnière et la rhinite perannuelle.

14. Utilisation selon la revendication 12, **caractérisée en ce que** le trouble des voies respiratoires est choisi parmi l'asthme allergique, l'asthme bronchique et la rhinite allergique.
